Europäisches Patentamt

European Patent Office

Office européen des brevets

(19)

(11) Veröffentlichungsnummer. **0 147 767**
**A2**

(12) # EUROPÄISCHE PATENTANMELDUNG

(21) Anmeldenummer: **84115675.5**

(22) Anmeldetag: **18.12.84**

(51) Int. Cl.⁴: **A 61 K 9/14**

(30) Priorität: **30.12.83 DE 3347579**

(43) Veröffentlichungstag der Anmeldung:
**10.07.85 Patentblatt 85/28**

(84) Benannte Vertragsstaaten:
**AT BE CH DE FR GB IT LI NL SE**

(71) Anmelder: **HOECHST AKTIENGESELLSCHAFT**
**Postfach 80 03 20**
**D-6230 Frankfurt am Main 80(DE)**

(72) Erfinder: **Hiller, Dietrich, Dr.**
**Riederbergstrasse 11**
**D-6200 Wiesbaden(DE)**

(72) Erfinder: **Korger, Gerhard, Dr.**
**Josef-Haydn-Strasse 32**
**D-6232 Bad Soden am Taunus(DE)**

(72) Erfinder: **Sellhorn, Kurt, Dr.**
**Loreleistrasse 30**
**D-6230 Frankfurt am Main 80(DE)**

(54) **Zubereitungen schwer wasserlöslicher Anthelminthika und Verfahren zu ihrer Herstellung.**

(57) Zubereitungen mit erhöhter Bioverfügbarkeit und Wirksamkeit von schwer wasserlöslichen Anthelminthika und Verfahren zur Herstellung dieser Zubereitungen werden beschrieben. Die Wirkstoffe weisen eine große spezifische Oberfläche auf.

EP 0 147 767 A2

HOECHST AKTIENGESELLSCHAFT    HOE 83/F 286    Dr.D/mk

Zubereitungen schwer wasserlöslicher Anthelminthika und Verfahren zu ihrer Herstellung

Die Anmeldung betrifft Zubereitungen mit erhöhter Bioverfügbarkeit und Wirksamkeit von schwer wasserlöslichen Anthelminthika und Verfahren zur Herstellung dieser Zubereitungen.

Es ist bekannt, daß oral applizierte Anthelminthika im Verdauungskanal resorbiert werden, in den Blutkreislauf und aus diesem in Organe übertreten und dort über längere Zeiträume ihre Wirksamkeit entfalten. Anthelminthika sind meist schwer wasserlösliche Substanzen, von denen bekannt ist, daß ihre biologische Wirksamkeit durch Vergrößerung der spezifischen Oberfläche gesteigert werden kann. Die größere spezifische Oberfläche verursacht eine höhere Lösungsgeschwindigkeit. Die Lösungsgeschwindigkeit aber ist häufig der die Resorption und, in ihrer Folge, die Wirksamkeit bestimmende Schritt. Der Zusammenhang von Korngröße und Wirksamkeit ist bereits beschrieben worden (W.A. Ritschel, Angewandte Biopharmazie, S. 293 ff, Wissenschaftliche Verlagsgesellschaft, Stuttgart (1973)).

Spezifische Oberflächen bis etwa 10 $m^2$/g lassen sich durch Trockenmahlung, wie z.B. Luftstrahlmahlung, gröberen Materials erzeugen. Ein anderes Verfahren zur Erreichung großer spezifischer Oberflächen ist die Fällung der Substanz, die sich z.B. in einem mit Wasser mischbaren Lösungsmittel in Lösung befindet, durch Zusatz von Wasser, Abtrennung des Sediments und gegebenenfalls Trocknung. Besitzt eine z.B. durch Fällung erhaltene Substanz eine spezifische Oberfläche von etwa 5 bis 10 $m^2$/g, so läßt sich diese durch Trockenmahlung nur noch unwesentlich erhöhen.

Versuche zeigten, daß wäßrige Suspensionen, enthaltend Anthelminthika vom Fenbendazol-Typ mit einer durch Trockenmahlung erhaltenen großen spezifischen Oberfläche ($5-10 \, m^2/g$ bestimmt mit der Gasadsorptionsmethode nach B.E.T.), eine höhere Wirksamkeit besitzen als solche enthaltend den Wirkstoff mit einer spezifischen Oberfläche von etwa $1 \, m^2/g$. Die visuell erkennbaren Wirkstoff-Teilchen in der erstgenannten Suspension waren größer, als aufgrund der spezifischen Oberfläche erwartet werden konnte.

Eine Vergrößerung der spezifischen Oberfläche schwer wasserlöslicher Anthelminthika durch modifizierte Fällungsmethoden bis etwa $25 \, m^2/g$ erbrachte eine weitere Steigerung der Wirksamkeit gegenüber Substanzen niedrigerer spezifischer Oberfläche. Die für die Fällung verwendeten Lösemittel müssen nach der Ausfällung quantitativ entfernt werden. Gemäß den Angaben in der Literatur sind mit spezifischen Oberflächen dieser Größenordnung die Möglichkeiten der Wirksamkeitssteigerung schwer wasserlöslicher Arzneistoffe ausgeschöpft. Ferner ist gemäß den Angaben in der Literatur die Abnahme der Partikelgröße technisch begrenzt. Kleinere Partikelgrößen als etwa 10 µm sind nur schwierig zu handhaben. Eine Partikelgröße von 10 µm entspricht aber nur einer spezifischen Oberfläche von größenordnungsmäßig $1 \, m^2/g$ (vgl. z.B. die o.g. Veröffentlichung von W.A. Ritschel).

Es wurde überraschenderweise gefunden, daß durch eine zusätzliche Naßvermahlung die biologische Wirksamkeit der schwer wasserlöslichen Anthelminthika maximiert werden kann, auch wenn diese Anthelminthika bereits eine durch Trockenmahlung oder Fällung erzeugte große spezifische Oberfläche besitzen.

- 3 -

Die Erfindung betrifft daher Zubereitungen von schwer wasserlöslichen Anthelminthika mit großer spezifischen Oberfläche erhalten durch Fällung und/oder Trockenmahlung und zusätzliche Naßmahlung. Sie zeichnen sich durch eine maximale Bioverfügbarkeit und Wirksamkeit aus.

Die Erfindung betrifft ferner ein Verfahren zur Herstellung dieser Zubereitungen, welches dadurch gekennzeichnet ist, daß man schwer wasserlösliche Anthelminthika in solche mit großer spezifischen Oberfläche überführt, indem man sie einer Fällung und/oder Trockenmahlung unterwirft, in Wasser oder einer Flüssigkeit, in welcher sich die Wirkstoffe schwer lösen, suspendiert und die Suspensionen zusätzlich naßvermahlt und gegebenenfalls anschließend trocknet. Der Naßmahleffekt wird durch vergleichende Korngrößenanalyse z.B. mit dem Coulter-Counter Gerät (Counter Electronics, Krefeld, Bundesrepublik Deutschland) kontrolliert.

Erfindungsgemäß kommen insbesondere schwerlösliche Anthelminthika folgender Substanzklassen in Betracht: Benzimidazol- und Benzthiazol-2-carbaminate (als Beispiele seien genannt: Fenbendazol, Albendazol, Oxibendazol, Oxfendazol, Parbendazol, Mebendazol, Flubendazol, Ciclobendazol, Tioxidazol oder substituierte Phenylsulfonyloxybenzimidazol-2-carbaminate, wie sie z.B. in der Deutschen Offenlegungsschrift 24 41 201 (=US-Patent 3.996.368) oder der deutschen Patentanmeldung P 32 47 615.9 beschrieben sind), andere anthelminthisch wirksame Benzimidazole (z.B. Triclabendazol, Cambendazol, Thiabendazol oder substituierte Phenylsulfonyloxybenzimidazole, wie sie in der Deutschen Offenlegungsschrift 31 32 167 (=Europäische Patentanmeldung mit der Veröffentlichungsnummer 72 532) beschrieben sind) oder anthelminthisch wirksame, sogenannte Benzimidazol-Prodrugs (Benzimidazol-Vorstufen) (z.B. Febantel, Thiophanat, substituierte Phenylguanidine, (vgl. z.B. Deutsche Offenlegungsschriften 26 08 238, 26 53 766 oder 28 36 385), Nitroverbindungen z.B. gemäß Deutscher Patentschrift 25 41 742 oder Ureidoverbindungen entsprechend der deutschen Patentanmeldung P 32 32 959.8).

Die erfindungsgemäßen Zubereitungen enthalten die genannten Anthelminthika alleine oder in Kombination miteinander oder mit weiteren Wirkstoffen. Eine bevorzugte Kombination ist z.B. Fenbendazol mit Triclabendazol.

Die Herstellung von Anthelminthika mit vergrößerter Oberfläche erfolgt durch Fällungsverfahren, wobei unter Fällungsverfahren sowohl Ausfällungs- als auch Kristallisationsverfahren verstanden werden. Hierzu können im Prinzip alle Kombinationen von den Wirkstoff lösenden mit den Wirkstoff nicht lösenden Solventien oder auch Lösemittelgemische verwendet werden.

Beispielhaft seien einige bevorzugte Kombinationen von Lösemitteln zur Kristallisation der Anthelminthika genannt:

Dimethylformamid, Dimethylsulfoxid, Acetonitril oder Dioxan jeweils in Verbindung mit Wasser oder Alkoholen, wie z.B. Methanol, Ethanol, Isopropanol, Amylalkohol, Methylglykol, oder organische Säuren, wie Eisessig, Propionsäure oder Trifluoressigsäure jeweils in Verbindung mit Wasser oder Alkoholen, wie Methanol, Ethanol, Isopropanol, Amylalkohol, Methylglykol. Je nach den Gegebenheiten können diese Lösemittel auch allein zur Kristallisation Verwendung finden.

Es ist ferner möglich, die Anthelminthika durch Salzbildung in einem geeigneten Lösemittel zu lösen und anschließend durch Neutralisation in der gewünschten Oberflächenbeschaffenheit wieder auszufällen.

Hierbei verfährt man z.B. wie folgt: Man löst die Substanz in Wasser oder einem Alkohol, wie z.B. Methanol, Ethanol, Isopropanol, Amylalkohol, Methylglykol, oder in Aceton oder Butanon

a) unter Zusatz von Säuren, wie z.B. wäßriger Salzsäure, HCl-Gas, Schwefelsäure, Salpetersäure, Phosphorsäure, Eisessig, Propionsäure, Trifluoressigsäure, oder

b) unter Zusatz von Basen, wie Natrium-, Kalium-, Calcium-, Bariumhydroxid, Natriummethylat, Natriumethylat, Natriumhydrid, Triethylamin, Pyridin, Ammoniak.

Anschließend neutralisiert man im Falle von a) z.B. mit einer unter b) genannten Base; im Falle von b) z.B. mit einer der unter a) genannten Säuren.

Die zur Verwendung gelangenden Säuren oder Basen können dabei entweder in reiner gasförmiger, flüssiger oder fester Form oder in Mischung bzw. Lösung mit einem geeigneten Solvens, wie z.B. Wasser, Methanol, Ethanol, Isopropanol, Amylalkohol, Methylglykol, Aceton, Butanon, eingesetzt werden.

Die Auswahl des optimalen Verfahrens richtet sich nach den dem Fachmann bekannten physikalischen und chemischen Gegebenheiten des verwendeten Wirkstoffs. Man arbeitet dabei z.B. bei tiefen oder hohen Temperaturen, bei einer genau definierten Rührgeschwindigkeit oder auch ohne Rühren und mit bestimmten Mengenverhältnissen der verwendeten Lösemittel untereinander und im Verhältnis zur eingesetzten Wirkstoffmenge.

Die Trockenmahlung ist bekannt und erfolgt z.B. mit Hammer-, Schlagkreuz-, Stift- oder Luftstrahlmühlen.

Bei der Naßmahlung werden Suspensionen des schwerlöslichen Wirkstoffs z.B. in Kugelmühlen, die um eine horizontale oder vertikale Achse rotieren, in Kugelmühlen, die auf einem Schwingtisch vibrieren oder in Rührwerkskugelmühlen, den sogenannten Perl- oder Sandmühlen, mit vertikaler oder horizontaler Achse gemahlen. Die Mühlen können aus unterschiedlichen Materialien bestehen, wie aus Hartglas, Hartporzellan oder Hartmetallen, und können im Chargen- oder Durchflußverfahren betrieben werden.

Als Suspensionsmedium eignen sich alle Flüssigkeiten, in denen die zu mahlende Substanz schwerlöslich ist. Aus praktischen Gründen wird Wasser bevorzugt.

Die Naßmahlung kann in Suspensionen erfolgen, die außer dem Wirkstoff bzw. dem Wirkstoff-Gemisch nur das Suspensionsmedium enthalten. Zweckmäßigerweise enthalten diese Suspensionen aber bereits einen oder mehrere Hilfsstoffe, wie zum Beispiel die folgenden:
Puffersubstanzen, wie Acetate, Phosphate oder Citrate,
Suspensionsstabilisatoren, wie Cellulose, Celluloseäther,
Polyvinylpyrrolidon oder hochdisperses Siliciumdioxid,
Netzmittel, wie Polyethylenglykolether oder Alkansulfonate,
Konservierungsmittel, wie Parabene, Sorbinsäure oder Benzylalkohol.

Das Suspensionsmedium beim Naßmahlen kann mit dem Applikationsmedium identisch sein, insbesondere dann, wenn der Wirkstoff in Form einer Suspension verabreicht werden soll. Es ist aber auch möglich, den oder die Wirkstoffe zum Beispiel in einer Fluorkohlenwasserstoffsuspension zu mahlen und das Suspensionsmittel dann für Applikationszwecke durch Verdampfen und Resuspendierung gegen Wasser auszutauschen.

Die Zubereitungen haben bevorzugt flüssige oder pastenförmige Konsistenz. Es sind aber auch feste Zubereitungen,
wie Pulver, Granulate, Kapseln oder Tabletten möglich.
Zu diesem Zweck wird den durch Naßmahlung gewonnenen Suspensionen das Suspensionsmedium durch geeignete Trockenmethoden, wie z.B. Verdampfen entzogen. Die Suspensionen
können auch auf Feststoffe, wie Siliciumdioxid, Maisstärke, Cellulose oder Lactose aufgezogen werden, worauf
danach ein Trocknen folgt. Die auf diese Weise gewonnenen,
die Anthelminthika in erfindungsgemäßer Form und gegebenenfalls Hilfsstoffe enthaltenden festen Zubereitungen
können entweder direkt oder nach weiteren Verfahrensschritten, wie Granulierung oder Tablettierung, bei denen
gegebenenfalls auch noch weitere übliche Hilfsstoffe zugesetzt werden, appliziert werden.

Die Zubereitungen gemäß der Erfindung sind wertvolle
Chemotherapeutika mit hoher Bioverfügbarkeit und Wirksamkeit und eignen sich zur Bekämpfung von parasitären
Erkrankungen von Mensch und Tier.

Sie sind besonders wirksam gegen eine große Anzahl von
Helminthen, z.B. Haemonchus, Trichostrongylus, Ostertagia, Cooperia, Metastrongyliden, Chabertia, Strongyloides, Oesophagostomum, Hyostrongylus, Ancylostoma,
Askariden und Heterakis, aber auch z.B. Fasciola und
Moniezia. Besonders ausgeprägt ist die Wirksamkeit
gegenüber Magen-Darmstrongyliden, Lungenwürmern, Bandwürmern und Leberegeln, von denen vor allem Haus- und
Nutztiere befallen werden. Deshalb werden die Zubereitungen gemäß der Erfindung außer am Menschen insbesondere in Tierarzneimitteln verwendet.

Die erfindungsgemäßen Zubereitungen werden wegen ihrer höheren Wirksamkeit in geringeren Dosierungen des Wirkstoffs als nach üblichen Verfahren hergestellte Zubereitungen verabreicht.

Die Konzentrationen der Wirkstoffe in erfindungsgemäßer Form in den damit hergestellten Präparaten liegen vorzugsweise für den Gebrauch als Veterinärarzneimittel zwischen 0,5 und 25 Gewichtsprozent; für den Gebrauch als Humanarzneimittel liegen die Konzentrationen der Wirkstoffe vorzugsweise zwischen 20 und 80 Gewichtsprozent.

Die Verfahrensprodukte sind nicht nur oral appliziert ausgezeichnet wirksam, sondern können auch parenteral appliziert werden.

Die Kombination der beiden Verfahrensschritte bringt gegenüber den einzelnen Schritten folgende erhebliche Vorteile:

1. Durch die vorgeschaltete Fällung oder Trockenmahlung wird die Naßmahlungsdauer erheblich verkürzt. Das Risiko, daß Abrieb der Mahlkörper in die Zubereitung gelangt, ist dadurch zu vernachlässigen.

2. Die Wirkstoffteilchen zeigen keine irreversible Agglomerisation.

3. Die Folge ist eine unerwartet hohe Wirksamkeit.

Die folgenden Beispiele beschreiben die Erfindung ohne sie einzuschränken.

Beispiel 1

630 g Fenbendazol werden bei Raumtemperatur in einem Gemisch aus 2000 g Methanol und 288 g 37%iger Salzsäure gelöst. Zu der angefallenen Lösung gibt man unter intensivem Rühren (®Ultra-Turrax, Fa. Janke & Kunkel) 354 g 33%ige Natronlauge. Das dabei ausgefällte Fenbendazol wird abfiltriert, mit entsalztem Wasser chloridfrei gewaschen und bei 70°C im Vakuum getrocknet. Ausbeute: 625 g. Spezifische Oberfläche 18,7 $m^2/g$.

Beispiel 2

a) Herstellung der Suspension

α) 25 g Fenbendazol (entnommen einem Ansatz von 550 kg Fenbendazol, das durch Fällung gemäß Beispiel 1 gewonnen wurde, spezifische Oberfläche 12,6 $m^2/g$) und 30 g hochdisperses Siliciumdioxid wurden in einer wäßrigen Lösung von 15 g Natriumcarboxymethylcellulose, 19 g Polyvinylpyrrolidon 25 000, 33 g Natriumcitrat x 2 $H_2O$, 1 g Citronensäure x $H_2O$, 1,7 g Methyl-4-hydroxybenzoat, 0,2 g Propyl-4-hydroxybenzoat und 5 g Benzylalkohol suspendiert. Mit Wasser wurde auf ein Endvolumen von 1,0 l aufgefüllt.

ß) Bei der Herstellung der Suspension wurde Fenbendazol gemäß Beispiel 1 eingesetzt.

b) Naßmahlung:

325 ml Suspension α oder ß wurden in eine Porzellankugelmühle von 1 l Inhalt gefüllt. Es wurden 0,5 l Porzellan-Kugeln mit einem Durchmesser von 8 - 10 mm zugegeben.

Die Porzellan-Kugelmühle wurde auf einer Schwingmühle <sup>R</sup>Vibratom der Fa. Siebtechnik, Mülheim/Ruhr, eingespannt und 150 Stunden gemahlen. Die Amplitude der Schwingungen betrug ca. 3 - 4 mm und ihre Frequenz, bedingt durch die Drehzahl des Elektromotors, ca. 1350.

Beispiel 3

Anstelle von Fenbendazol wurden 25 g 5-(4-Fluorphenyl-sulfonyloxy)-benzimidazol-2-carbaminsäure-methylester

α) gewonnen durch Fällung, spezifische Oberfläche 5,4 $m^2$/g; (Die Fällung wurde wie folgt durchgeführt: 7.3 kg Anthelminthikum wurden unter Rühren in 80 l Methanol und 1,95 l Salzsäure (konz.) gelöst. Die Lösunge wurde zwischen 10 - 20°C mit 30 %iger wäßriger Natronlauge auf pH 7 bis 7,5 eingestellt. Das aufgefallene Produkt wurde abgetrennt, mit einem Methanol-Wasser-Gemisch gewaschen und getrocknet. 25 g des Produktes wurden zur Herstellung der Suspension entnommen.)

ß) gewonnen durch Trockenmahlung (Luftstrahlmahlung) von 3α, spezifische Oberfläche 8,2 $m^2$/g;

γ) gewonnen durch Trockenmahlung (Stiftmühlenmahlung) von 3α, spezifische Oberfläche 6,9 $m^2$/g;

δ) gewonnen durch Fällung, spezifische Oberfläche 2,5 $m^2$/g; (Die Fällung wurde wie folgt durchgeführt: 75 g Anthelminthikum wurden in 300 ml Dimethylformamid heiß gelöst und mit 1000 ml Methanol versetzt. Beim Abkühlen fiel das Produkt aus. Es wurde mit einem Gemisch aus Methanol und Diisopropylether gewaschen. 25 g des Produktes wurden zur Herstellung der Suspension entnommen.) und

ε) gewonnen durch Fällung, spezifische Oberfläche 2 4,8 $m^2$/g; (Die Fällung wurde wie folgt durchgeführt: 25 g Anthelminthikum wurden in 250 ml Dioxan unter Erwärmen und kräftigem Rühren gelöst. Danach wurden 2000 ml Wasser tropfenweise zugesetzt. Das ausgefallene Produkt wurde abgetrennt und mit einem Gemisch aus Wasser und Methanol gewaschen.)
eingesetzt. Ansonsten wurde wie in Beispiel 2 a) α) oder 2 a) α) + 2 b) angegeben, verfahren.

## Beispiel 4

## Tierversuch

### Behandlung:

Jeweils 3 Schafen wurde eine der Zubereitungen A, B, C bzw. D oral verabreicht. Die Wirkstoffkonzentration der Zubereitung betrug 2,5 Gewichtsprozent und die Dosierung 1x6 mg Fenbendazol/kg Körpermasse.

### Zubereitungen:

A: Suspension entsprechend Beispiel 2 a) α), spezifische Oberfläche des Fenbendazols: 12,6 $m^2$/g, ohne Naßmahlung.

B) Suspension entsprechend Beispiel 2 a) α) mit Naßmahlung gemäß Beispiel 2 b).

C) Suspension entsprechend Beispiel 2 a) ß), spezifische Oberfläche des Fenbendazols 18,7 $m^2$/g, ohne Naßmahlung

D) Suspension entsprechend Beispiel 2 a) ß) mit Naßmahlung gemäß Beispiel 2 b).

### Auswertung:

Die Blutentnahme erfolgte fließend aus der Vena jugularis vor Präparat-Applikation sowie 1, 2, 4, 6, 8, 24, 30, 48, 54, 72, 96, 120, 144, 168, 240 und 336 Stunden post appl.;

Die Proben wurden bei -23°/-24°C tiefgefroren bis zur Untersuchung aufbewahrt.

- 12 -                    **0147767**

Die hochdruckflüssigkeitschromatographische Untersuchung der
Serumspiegel erfolgte auf Fenbendazol und die Metaboliten SO-Fenbendazol, $SO_2$-Fenbendazol sowie p-HO-
Fenbendazol.

Die Meßdaten sind in Tabelle 1 aufgeführt. Sie wurden
gruppenweise arithmetisch gemittelt. Vor Applikation waren die Serumspiegel O.

Tabelle 1

Serumspiegel von Fenbendazol, SO- und $SO_2$-Fenbendazol nach oraler Verabreichung der Zubereitungen A, B, C und D in ng/ml

| Zeit in Stunden p. appl. | Fenbendazol | | | | SO-Fenbendazol | | | | $SO_2$-Fenbendazol | | | |
|---|---|---|---|---|---|---|---|---|---|---|---|---|
| | A | B | C | D | A | B | C | D | A | B | C | D |
| 1 | 0 | 15,3 | 0 | 15 | 0 | 0 | 0 | 0 | 0 | 0 | 0 | 0 |
| 2 | 14 | 61 | 19 | 77,3 | 0 | 15,3 | 0 | 14,3 | 0 | 0 | 0 | 0 |
| 4 | 36 | 127,6 | 64,6 | 204,3 | 8,3 | 57,3 | 19,3 | 81,3 | 0 | 0 | 3,3 | 0 |
| 6 | 60 | 199,6 | 110,3 | 265,6 | 34,6 | 148,6 | 72,3 | 174,6 | 0 | 4,3 | 5,6 | 6,3 |
| 8 | 53 | 197,6 | 107,6 | 249 | 52,6 | 106 | 102,3 | 235 | 0 | 9 | 11,6 | 19,3 |
| 24 | 86,6 | 203,3 | 142 | 285,6 | 140,3 | 350 | 257,6 | 454 | 27,3 | 81,3 | 63,3 | 100,6 |
| 30 | 90,6 | 164,3 | 115,6 | 254 | 165,6 | 358,3 | 261 | 477 | 39 | 106,6 | 82 | 126,3 |
| 48 | 49,6 | 82 | 66,3 | 121,6 | 91,3 | 170 | 130,6 | 227,6 | 43,6 | 103,3 | 76 | 113 |
| 54 | 45,3 | 62,3 | 65,6 | 103,6 | 86,3 | 144 | 112,3 | 191 | 44 | 104,3 | 75 | 103,6 |
| 72 | 20,6 | 30,3 | 22,6 | 50,6 | 46,3 | 69 | 52 | 98,3 | 35,6 | 70,6 | 60 | 79,6 |
| 96 | 0 | 8,3 | 4 | 11 | 10,3 | 20,3 | 11,3 | 26,6 | 25,6 | 49,6 | 39 | 49,6 |
| 120 | 0 | 3,6 | 0 | 0 | 0 | 5 | 0 | 4,3 | 15,6 | 32 | 22,3 | 31,3 |
| 144 | 0 | 0 | 0 | 0 | 0 | 0 | 0 | 0 | 9,3 | 17,6 | 20,3 | 18,6 |
| 168 | 0 | 0 | 0 | 0 | 0 | 0 | 0 | 0 | 4 | 11 | 9 | 10,3 |
| 240 | | | | | | | | | 0 | 0 | 3,6 | 0 |
| 336 | | | | | | | | | 0 | 0 | 0 | 0 |

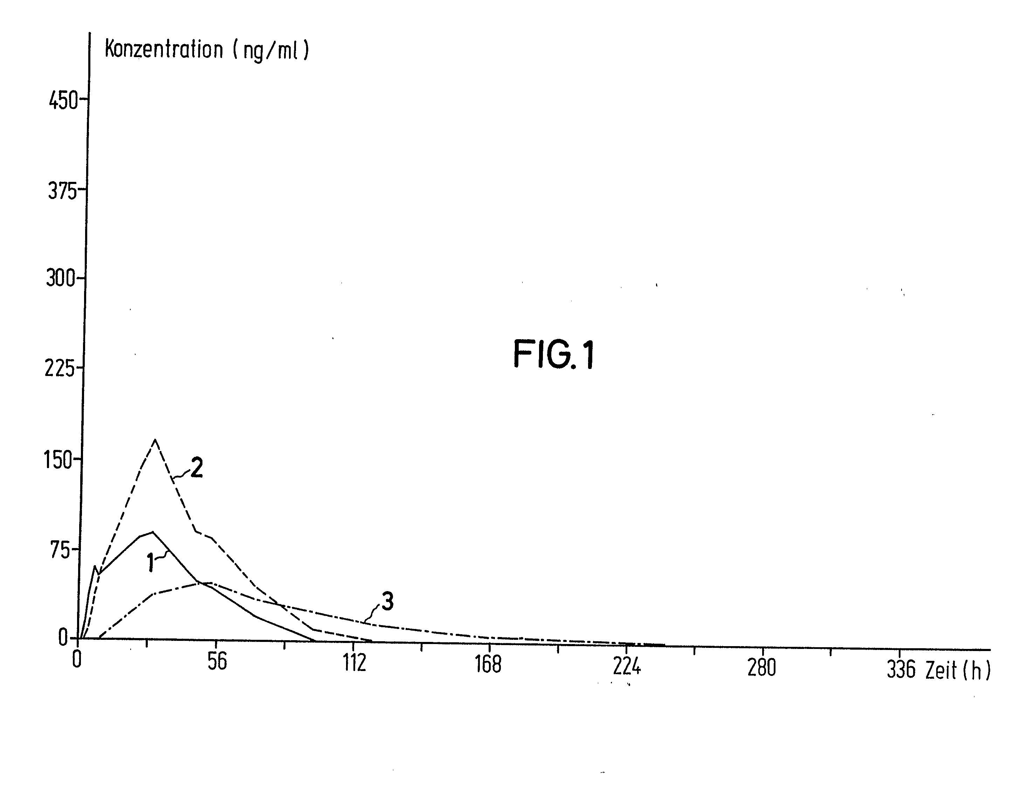

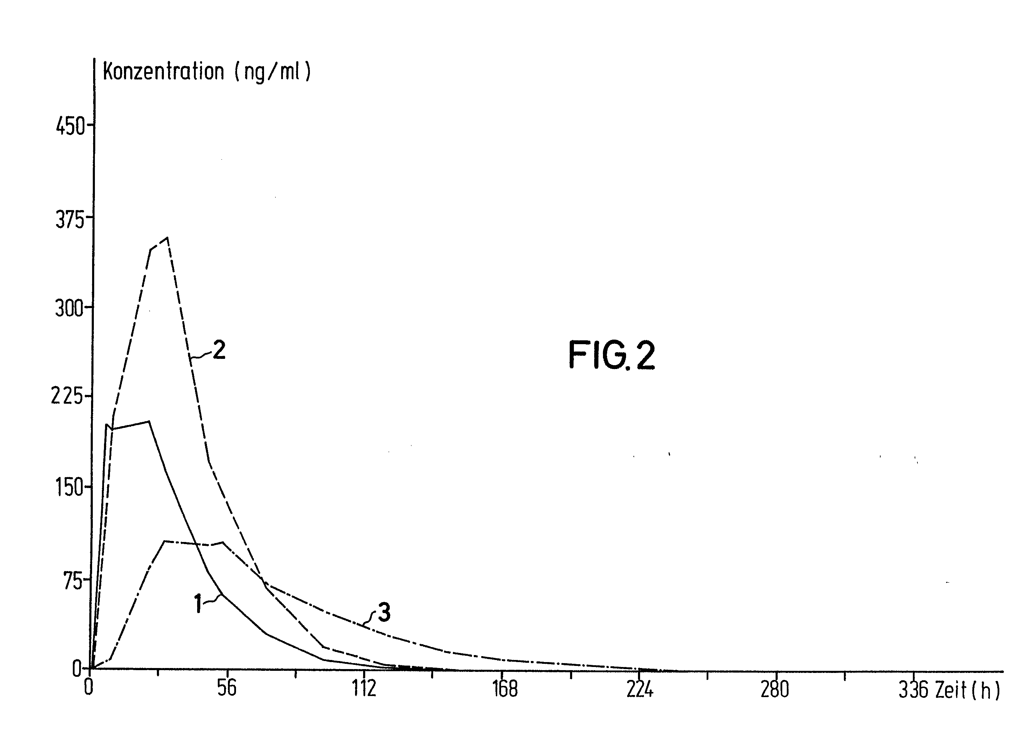

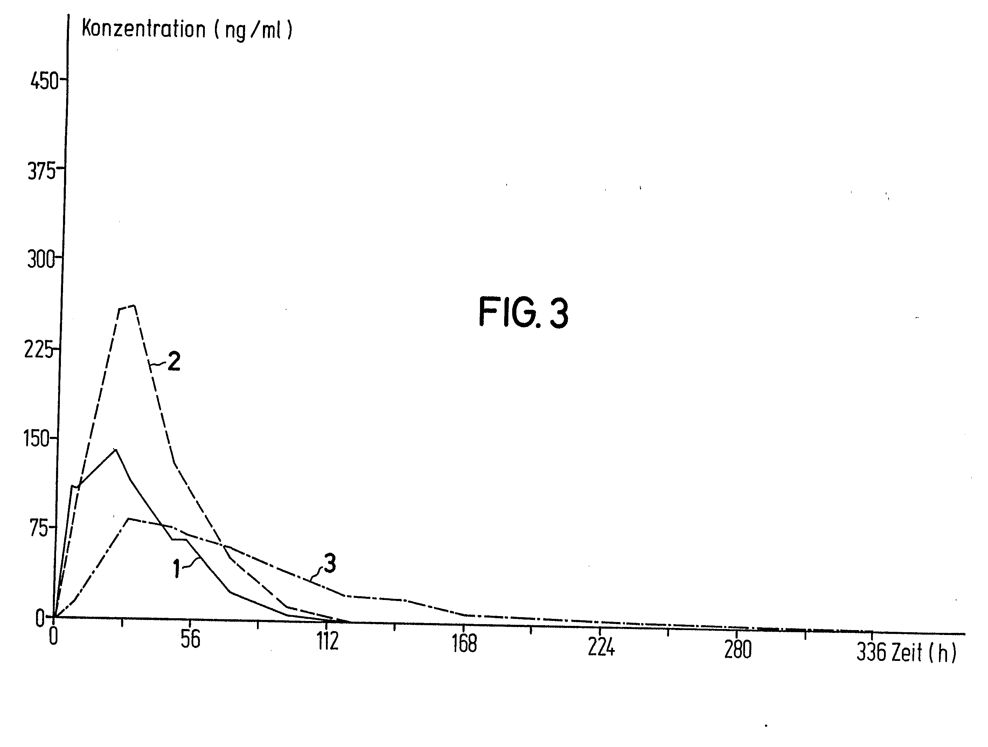

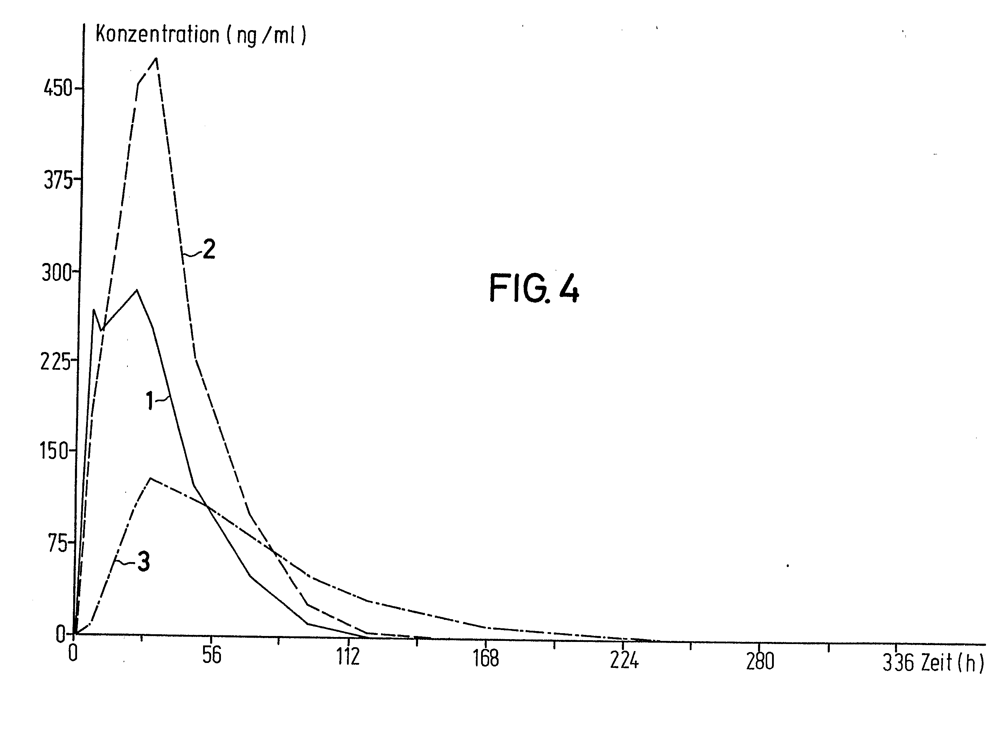

Die für Fenbendazol (Kurve 1), SO-Fenbendazol (Kurve 2) und SO$_2$-Fenbendazol (Kurve 3) gemessenen (arithmetisch gemittelten) Serumspiegel in ng/ml sind den Figuren 1-4 zu entnehmen. (Figur 1 stellt die Serumspiegel nach Verabreichung von Präparat A, Figur 2 von Präparat B, Figur 3 von Präparat C und Figur 4 von Präparat D dar). Der Metabolit p-HO-Fenbendazol konnte in keiner der Proben nachgewiesen werden.

Ergebnis:

Der Vorteil der höheren spezifischen Oberfläche des Wirkstoffs ergibt sich aus dem Vergleich C gegen A, der unerwartet hohe Einfluß der Naßmahlung aus dem Vergleich B/D gegen A/C. Auch die Metaboliten von Fenbendazol unterliegen einem analogen Verhalten von Resorption und Resorptionsgeschwindigkeit.

Beispiel 5

Tierversuch

Behandlung:

Jeweils 3 Schafen wurde eine der Zubereitungen A, B, C, D, E bzw. F in einer Dosierung von 1x6 mg 5-(4 Fluorphenylsulfonyloxy)-benzimidazol-2-carbaminsäure-methylester pro kg Körpermasse p.o. verabreicht. Die Zubereitungen G und H wurden in den Dosierungen von 1x4,5 und 1x3,0 mg/kg KM appliziert. Die Wirkstoffkonzentration war in allen Fällen 2,5 Gewichtsprozent.

Zubereitungen:

A: Suspension entsprechend Beispiel 3α) bzw. 2a); spezifische Oberfläche des Wirkstoffs 5,4 m$^2$/g, ohne Naßmahlung.

B: Suspension mit Naßmahlung entsprechend Beispiel 3α) bzw. 2a)+b).

C: Suspension entsprechend Beispiel 3ß) bzw. 2a); spezifische Oberfläche des Wirkstoffs 8,2 m$^2$/g, ohne
Naßmahlung.

D: Suspension entsprechend Beispiel 3γ) bzw. 2a); spezifische Oberfläche des Wirkstoffs 6,9 m$^2$/g, ohne
Naßmahlung.

E: Suspension entsprechend Beispiel 3δ) bzw. 2a), spezifische Oberfläche des Wirkstoffs 2,5 m$^2$/g, ohne
Naßmahlung.

F: Suspension mit Naßmahlung entsprechend Beispiel 3ß)
bzw. 2a)+b).

G: Suspension entsprechend Beispiel 3ε) bzw. 2a); spezifische Oberfläche des Wirkstoffs 24,8 m$^2$/g, ohne
Naßmahlung.

H: Suspension mit Naßmahlung entsprechend Beispiel 3ε)
bzw. 2a)+b).

<u>Auswertung</u>:

Die Blutentnahme erfolgte fließend aus der Vena jugularis
vor Präparat-Applikation sowie 2, 4, 6, 8, 24, 30, 48,
54, 72, 96, 120, 144 und 168 Stunden post appl.;

Die Proben wurden bei -23°/-24°C tiefgefroren bis zur
Untersuchung aufbewahrt. Durch hochdruckflüssigkeitschromatographische Untersuchung wurde der Serumspiegel von
5-(4-Fluorphenylsulfonyloxy)-benzimidazol-2-carbaminsäure-
methylester bestimmt.

Die Meßdaten (arithmetische Mittel) sind in der Tabelle 2
aufgeführt. Vor Applikation waren die Werte 0.

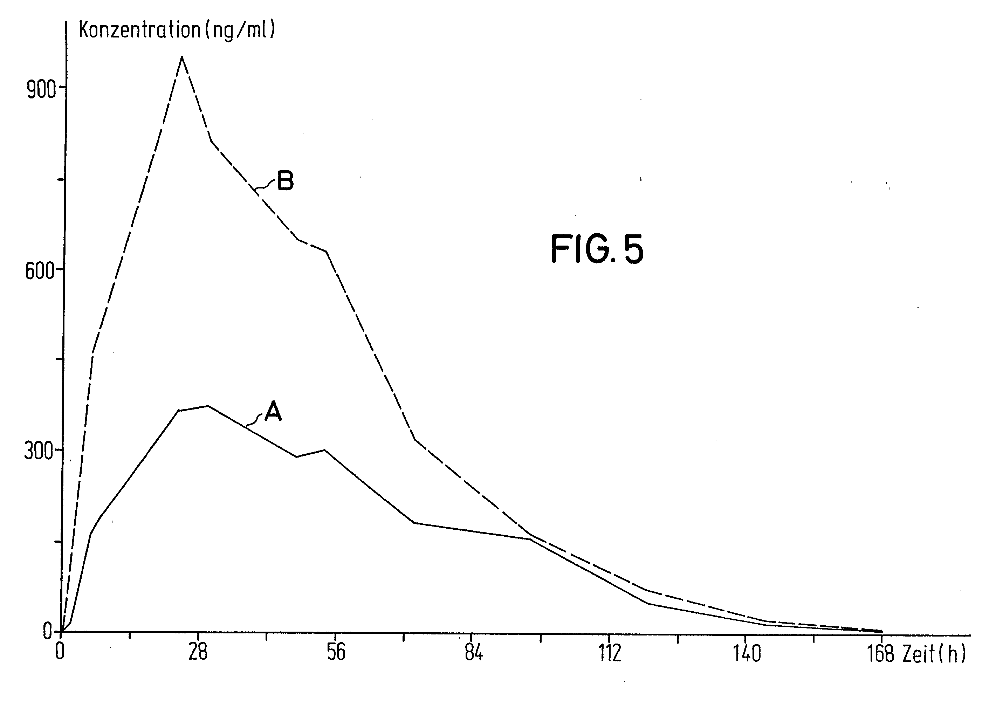

Der jeweilige Serumspiegel (arithmetischer Mittelwert in ng/ml) ist den Figuren 5-9 zu entnehmen. Die Zuordnung der Figuren zu den Präparaten A bis H ist wie folgt:

Figur 5 {Suspension A - Kurve A
{Suspension B - Kurve B

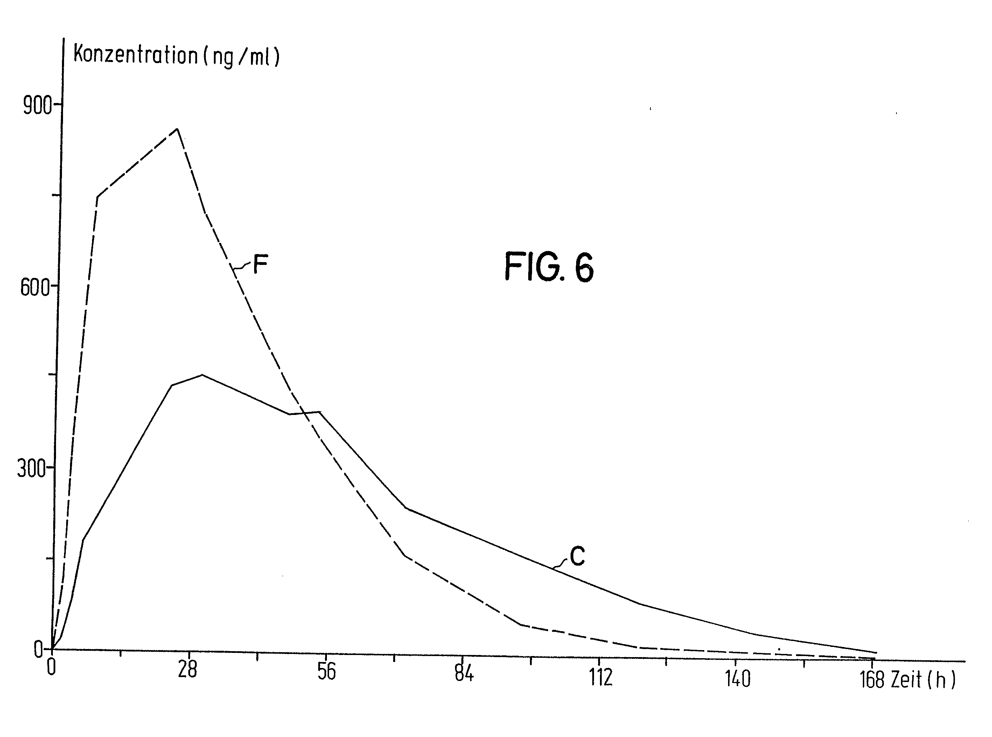

Figur 6 {Suspension C - Kurve C
{Suspension F - Kurve F

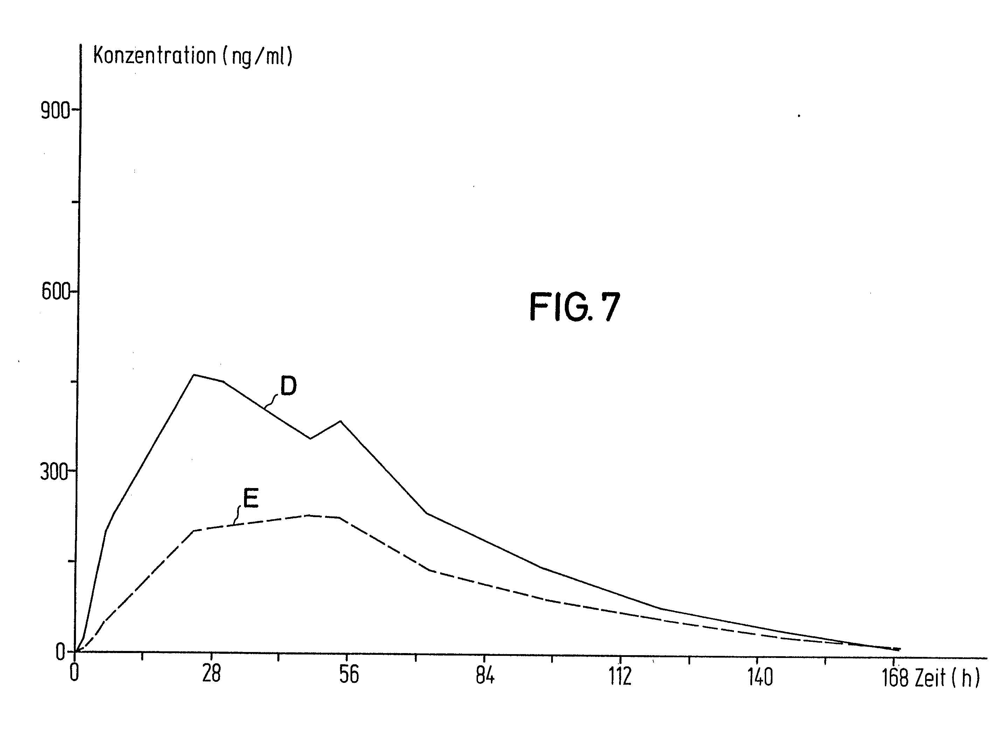

Figur 7 {Suspension D - Kurve D
{Suspension E - Kurve E

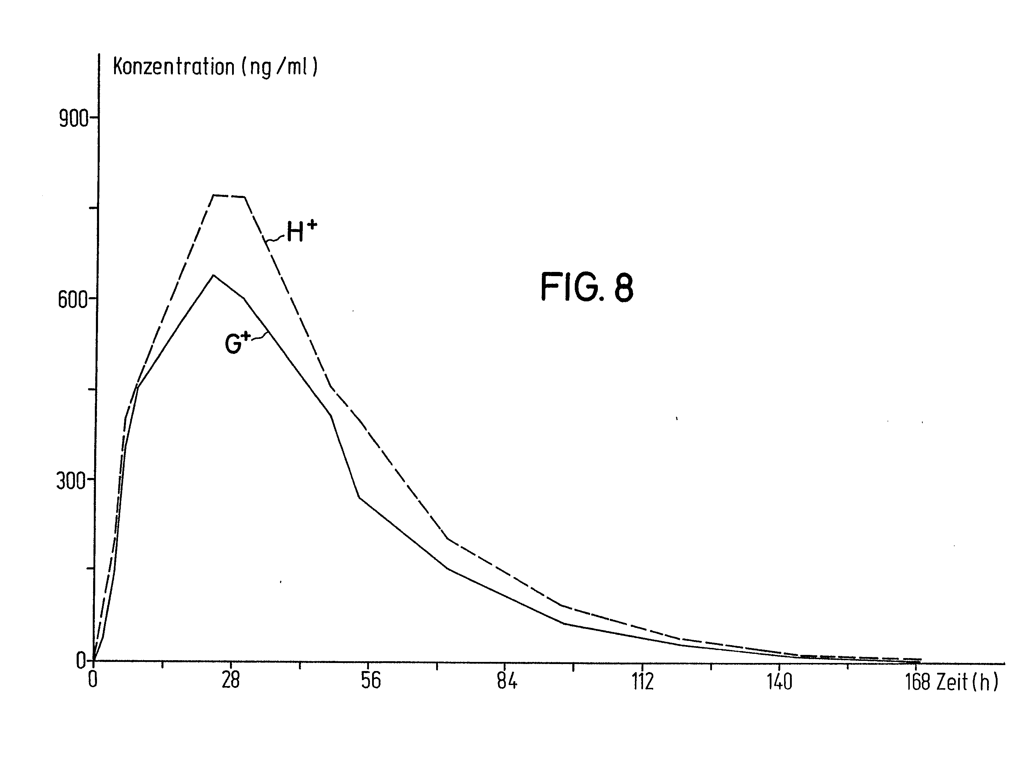

Figur 8 {Suspension G, Dosis 3 mg - Kurve G*
{Suspension H, Dosis 3 mg - Kurve H*

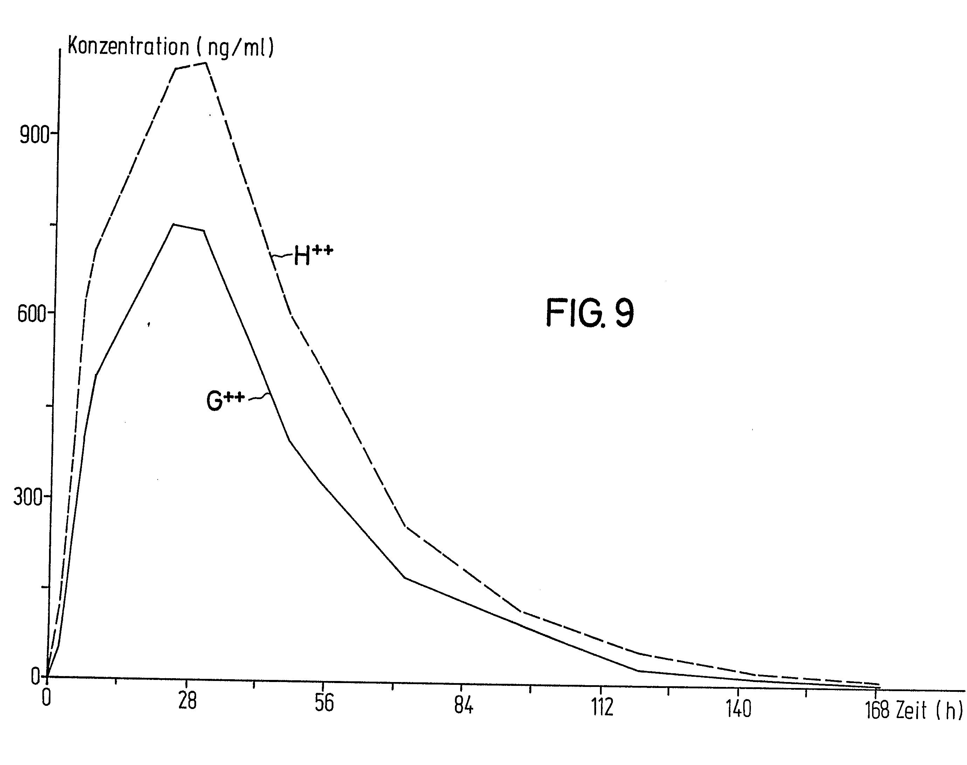

Figur 9 {Suspension G, Dosis 4,5 mg - Kurve G**
{Suspension H, Dosis 4,5 mg - Kurve H**

Ergebnis:

Die Zubereitungen enthaltend den Wirkstoff mit höherer spezifischer Oberfläche zeigen auch höhere Blutspiegel, wie sich aus der Reihe der Zubereitungen E, A, D, C und G ergibt. Eine wesentliche Erhöhung der Blutspiegel wurde im Vergleich zu den Präparatent A, C und G nach Verabreichung der Präparate B, F und H, die zusätzlich naßgemahlen worden waren, beobachtet.

Tabelle 2

Serumspiegel von 5-(4-Fluorphenyl-sulfonyloxyl)-benzimidazol-2-carbaminsäure-
methylester nach oraler Verabreichung der Zubereitungen A bis H in ng/ml

| Zeit in Stunden p. appl. | A | B | C | D | E | F | G* | H* | G** | H** |
|---|---|---|---|---|---|---|---|---|---|---|
| 2 | 17,3 | 114,7 | 19,7 | 23,0 | 7,0 | .125 | 43 | 83 | 56 | 117 |
| 4 | 76,0 | 268,3 | 87,7 | 105,3 | 22,7 | 347 | 146 | 207 | 179 | 358 |
| 6 | 165,0 | 452,3 | 179,7 | 200,0 | 56,3 | 599 | 350 | 400 | 389 | 624 |
| 8 | 189,0 | 507,3 | 209,3 | 233,7 | 71,0 | 744 | 453 | 454 | 503 | 709 |
| 24 | 365,0 | 950,0 | 434,0 | 461,3 | 196,3 | 865 | 640 | 776 | 751 | 1007 |
| 30 | 372,7 | 813,0 | 455,7 | 450,3 | 203,3 | 722 | 600 | 775 | 742 | 1018 |
| 48 | 292,7 | 654,0 | 388,0 | 357,0 | 228,0 | 428 | 412 | 457 | 390 | 593 |
| 54 | 301,0 | 623,0 | 392,3 | 386,3 | 224,0 | 354 | 274 | 396 | 335 | 521 |
| 72 | 171,3 | 314,7 | 240,7 | 227,7 | 139,7 | 160 | 156 | 195 | 170 | 257 |
| 96 | 152,0 | 164,0 | 162,0 | 142,3 | 96,3 | 57 | 64 | 87 | 87 | 121 |
| 120 | 53,0 | 70,7 | 93,0 | 81,7 | 62,0 | 17 | 27 | 38 | 25 | 56 |
| 144 | 19,3 | 26,7 | 42,3 | 43,3 | 30,3 | 7 | 12 | 15 | 8 | 26 |
| 168 | 7,7 | 9,7 | 17,7 | 15,0 | 17,3 | 2 | 5 | 8 | 4 | 13 |

\*   Dosierung: 3 mg/kg KM

\*\* Dosierung: 4,5 mg/kg KM

Patentansprüche:

1. Zubereitungen schwer wasserlöslicher Anthelminthika dadurch gekennzeichnet, daß die Wirkstoffe eine große spezifische Oberfläche, erzeugt durch Fällungs- und/oder Trockenmahlverfahren und zusätzliche Naßmahlung, aufweisen.

2. Zubereitungen gemäß Anspruch 1, dadurch gekennzeichnet, daß sie als Anthelminthika Benzimidazol-2-carbaminate, Benzthiazol-2-carbaminate, Benzimidazole oder Benzimidazol-Prodrugs mit großer spezifischer Oberfläche enthalten.

3. Zubereitungen gemäß Anspruch 2, dadurch gekennzeichnet, daß die Zubereitungen ein oder mehrere Anthelminthika mit großer spezifischer Oberfläche enthalten.

4. Zubereitungen gemäß Anspruch 2, dadurch gekennzeichnet, daß sie Fenbendazol, 5-(4-Fluorphenylsulfonyloxy benzimidazol-2-carbaminsäure-methylester und/oder Triclabendazol enthalten.

5. Verfahren zur Herstellung der Zubereitungen gemäß Anspruch 1, dadurch gekennzeichnet, daß man schwerwasserlösliche Anthelminthika in solche mit großer spezifischer Oberfläche überführt, indem man sie einer Fällung und/oder Trockenmahlung unterwirft, in Wasser oder einer Flüssigkeit, in welcher sich die Wirkstoffe schwer lösen, suspendiert und die Suspensionen zusätzlich naßvermahlt und gegebenenfalls anschließend trocknet.

6. Verfahren gemäß Anspruch 5, dadurch gekennzeichnet, daß das Suspensionsmittel Wasser ist und noch weitere Hilfsstoffe enthält.

Patentansprüche für Österreich

1. Verfahren zur Herstellung von Zubereitungen schwer wasserlöslicher Anthelminthika, dadurch gekennzeichnet, daß man schwerwasserlösliche Anthelminthika in solche mit großer spezifischer Oberfläche überführt, indem man sie einer Fällung und/oder Trockenmahlung unterwirft, in Wasser oder einer Flüssigkeit, in welcher sich die Wirkstoffe schwer lösen, suspendiert und die Suspensionen zusätzlich naßvermahlt und gegebenenfalls anschließend trocknet.

2. Verfahren gemäß Anspruch 1, dadurch gekennzeichnet, daß das Suspensionsmittel Wasser ist und noch weitere Hilfsstoffe enthält.

3. Verfahren gemäß Anspruch 1, dadurch gekennzeichnet, daß als Anthelminthika Benzimidazol-2-carbaminate, Benzthiazol-2-carbaminate, Benzimidazole oder Benzimidazol-Prodrugs mit großer spezifischer Oberfläche verwendet werden.

4. Verfahren gemäß Anspruch 3, dadurch gekennzeichnet, daß ein oder mehrere Anthelminthika mit großer spezifischer Oberfläche verwendet werden.

5. Verfahren gemäß Anspruch 3, dadurch gekennzeichnet, daß man Fenbendazol, 5-(4-Fluorphenylsulfonyloxy)-benzimidazol-2-carbaminsäure-methylester und/oder Triclabendazol einsetzt.

Konzentration ( ng/ml )

FIG.1

1/9

0147767

FIG. 2

FIG. 3

FIG. 4

FIG. 5

0147767

FIG. 6

0147767

FIG. 7

FIG. 8

0147767

FIG. 9

0147767